# EUROPÄISCHE PATENTSCHRIFT

(11) **EP 1 204 765 B1**
(45) Veröffentlichungstag und Bekanntmachung des Hinweises auf die Patenterteilung: **28.09.2005**
(21) Anmeldenummer: 00958186.9
(22) Anmeldetag: 25.07.2000
(51) Int. Cl.: C12Q 1/68

(54) **VERFAHREN ZUR RELATIVEN QUANTIFIZIERUNG DER METHYLIERUNG VON CYTOSIN BASEN IN DNA-PROBEN**
METHOD FOR RELATIVE QUANTIZATION OF METHYLATION OF CYTOSIN-TYPE BASES IN DNA SAMPLES
PROCEDE DE QUANTIFICATION RELATIVE DE LA METHYLATION DES BASES DU TYPE CYTOSINE DANS DES ECHANTILLONS D'ADN

(30) Priorität: 26.07.1999 DE 19935772
(43) Veröffentlichungstag der Anmeldung: 15.05.2002
(73) Patentinhaber: Epigenomics AG, 10178 Berlin (DE)
(72) Erfinder: BERLIN, Kurt, D-14532 Stahnsdorf (DE)
(74) Vertreter: Schubert, Klemens
(86) Internationale Anmeldenummer: PCT/DE2000/002490
(87) Internationale Veröffentlichungsnummer: WO 2001/007647

(56) Entgegenhaltungen:
- WO-A-98/56952
- DE-A- 4 212 939
- DE-A- 19 754 482
- US-A- 5 837 832
- OLEK A ET AL: "A modified an improved method for bisulphite based cytosine methylation analysis" NUCLEIC ACIDS RESEARCH,GB,OXFORD UNIVERSITY PRESS, SURREY, Bd. 24, Nr. 24, 1996, Seiten 5064-5066, XP002106408 ISSN: 0305-1048

## Beschreibung

Die Erfindung betrifft ein Verfahren zur relativen Quantifizierung der Methylierung von Cytosin Basen in DNA-Proben.

5-Methylcytosin ist die häufigste kovalent modifizierte Base in der DNA eukaryotischer Zellen. Sie spielt beispielsweise eine Rolle in der Regulation der Transkription, genomischem Imprinting und in der Tumorgenese. Die Identifizierung von 5-Methylcytosin als Bestandteil genetischer Information ist daher von erheblichem Interesse. 5-Methylcytosin-Positionen können jedoch nicht durch Sequenzierung identifiziert werden, da 5-Methylcytosin das gleiche Basenpaarungsverhalten aufweist wie Cytosin. Darüberhinaus geht bei einer PCR-Amplifikation die epigenetische Information, welche die 5-Methylcytosine tragen, vollständig verloren.

Es sind mehrere Verfahren bekannt, die diese Probleme lösen. Meist wird eine chemische Reaktion oder enzymatische Behandlung der genomischen DNA durchgeführt, infolge derer sich die Cytosin von den Methylcytosin Basen unterscheiden lassen. Eine gängige Methode ist die Umsetzung von genomischer DNA mit Bisulfit, die nach alkalischer Hydrolyse in zwei Schritten zu einer Umwandlung der Cytosin Basen in Uracil führt (Shapiro, R., Cohen, B., Servis, R. Nature 227, 1047 (1970). 5-Methylcytosin bleibt unter diesen Bedingungen unverändert. Die Umwandlung von C in U führt zu einer Veränderung der Basensequenz, aus der sich durch Sequenzierung nun die ursprünglichen 5-Methylcytosine ermitteln lassen (nur diese liefern noch eine Bande in der C-Spur).

Eine Übersicht über die weiteren bekannten Möglichkeiten, 5-Methylcytosine nachzuweisen, ist mitsamt der dazugehörigen Literatur dem folgenden Übersichtsartikel zu entnehmen: Rein, T., DePamphilis, M. L., Zorbas, H., Nucleic Acids Res. 26, 2255 (1998).

Bei dem in DE 197 54 482 A1 beschriebenen Verfahren erhält man nicht unmittelbar Kenntnis über die Methylierungsrate in einem Fragment, vielmehr erhält man zunächst ein abstraktes Muster, dessen Komponenten nicht notwendigerweise den dafür ursächlichen Fragmenten zugeordnet werden müssen

Paul, C.L et al. (Biotechniques (1996) 21 (1) 126-33: Cytosin methylation: quantitation by automated genomic sequencing and GENESCAN analysis) beschreibt eine Methode des Sequenzierens unter Verwendung von unterschiedlich markierten Thymin und Cytosinbasen. Vorliegend sind aber weder genomisches Sequenzieren noch überhaupt eine E-lektrophoresemethode involviert.

### Yurov, Y. B. et al. (Human Genetics (1996) 97 (3) 390-8:

High resolution multicolor fluorescence in situ hybridization using cyanine and fluorescein dyes: rapid chromosome identification by directly fluorescently labelled alphoid DNA probes) betrifft die Verwendung von Cy3 und Cy5-dCTP zu Markierungszwecken.

US-A-5 837 832 beschreibt Arrays von Oligonukleotiden und deren Hybridisierung an Proben-DNA. Jedoch werden weder DNA-Methylierung detektiert noch eignen sich die dort beschriebenen Arrays zum Hybridisieren unterschiedlicher Fragmente aus komplexen Amplifikationen derart, dass sie zu den Primern komplementäre Oligonukleotide enthalten und damit je Oligonukleotid spezifisch ein Fragment binden

Im Stand der Technik sind verschiedene Verfahren bekannt, mit denen man Oligonukleotid-Arrays herstellen kann. Sie lassen sich grob in 3 Gruppen einteilen:
1) Alle Oligomere werden auf herkömmliche Weise einzeln und in relativ großer Menge in speziellen Syntheseautomaten hergestellt und danach einzeln auf den Träger aufpipettiert. Dazu verwendet man üblicherweise automatische, hochpräzise Mikropipettierroboter. Der Vorteil dieses Verfahrens ist, dass es weitgehend auf bereits optimierten Standardmethoden und -Geräten beruht. Dadurch kann man qualitativ hochstehende DNA-Arrays mit mit sehr reinen Oligomeren herstellen, was einen äußerst positiven Einfluß auf die mit dem Array erzielbare Detektionsempfindlichkeit und -Zuverlässigkeit hat. Der große Nachteil des Verfahrens ist, dass es enorm aufwendig und deshalb teuer ist.
2) Die Oligomere werden durch Pipettieren in kleinsten Mengen direkt auf dem Substrat synthetisiert. Auf jedem Rasterpunkt wird die dort vorgesehene Oligomerkette Nukleobase für Nukleobase aufgebaut. Zur Pipettierung verwendet man ähnlich wie bei Verfahren 1) einen spezialisierten Mikropipettierroboter oder z. B. eine Vorrichtung, die Kanäle zur Zuführung der einzelnen Synthesebausteine zu den jeweiligen Punkten des Arrays enthält (EP-A-0915897). Das chemische Syntheseverfahren ist grundsätzlich das gleiche wie bei der herkömmlichen Oligomer-Synthese im Syntheseautomaten.
3) Die Oligomere werden wie bei Methode 2) direkt auf dem Substrat synthetisiert, die gezielte Anbindung der richtigen Nukleobasen an den richtigen Rasterpunkten geschieht jedoch durch eine vollkommen parallele, aus der Halbleiterfertigung stammende photolithographische Technik anstelle von seqenziellen, zielgenauen Pipettierschritten. Das Verfahren basiert darauf, dass man mit Licht einer bestimmten Wellenlänge gezielt die 5'-OH Schutzgruppen von Oligonukleotiden entfernen kann. Durch geeignete örtliche Bestrahlungsmuster kann man somit Oligonukleotid-Enden an genau jenen Rasterpunkten reaktionsfähig machen, an die man im nächsten Schritt einen neuen Nukleotidbaustein anbinden will. Bei vollständiger Benetzung der Arrayoberfläche mit einer Nukleotidbaustein-Lösung wird somit nur an den vorher belichteten Stellen eine Nukleobase angebunden, alle unbelichteten Stellen bleiben unverändert. Die örtlichen Belichtungsmuster werden erzeugt, indem man eine mikrophotographische schwarzweiß Maske zwischen dem Substrat und der Lichtquelle positioniert, die alle Rasterstellen abdeckt, die nicht reaktionsfähig gemacht werden sollen.

Diese Methode ist wegen der hohen Parallelität in der Verarbeitung sehr schnell und effizient, zudem ist sie wegen der hohen Präzision, die mit Photolithographie erreicht werden kann, gut dazu geeignet, sehr hohe Rasterdichten zu erzielen.

Eine Übersicht über den Stand der Technik in der Oligomer Array Herstellung läßt sich auch einer im Januar 1999 erschienen Sonderausgabe von Nature Genetics (Nature Genetics Supplement, Volume 21, January 1999) und der dort zitierten Literatur entnehmen.

Stand der Technik, welcher sich allgemein auf die Verwendung von Oligomer Arrays und photolithographisches Maskendesign beziehen, ist z. B. US 5,837,832, US 5,856,174, WO98/27430 und US 5,856,101.

Die Amplifikation von DNA mittels PCR ist Stand der Technik.

Aufgabe der vorliegenden Erfindung ist es daher ein Verfahren zur relativen Quantifizierung von Cytosin-Methylierungen in genomischen DNA Proben zu schaffen, welches die Nachteile des Standes der Technik Überwindet.

Die Aufgabe wird dadurch gelöst, dass ein Verfahren zur relativen Quantifizierung der Methylierung von Cytosin Basen in DNA-Proben zur Verfügung gestellt wird, wobei man folgende Verfahrensschritte ausführt:
a) man setzt eine genomische DNA-Probe mit einem Reagenz chemisch um, wobei 5-Methylcytosin und Cytosin unterschiedlich reagieren und diese somit nach der Reaktion ein unterschiedliches Basenpaarungsverhalten in der DNA Duplex zeigen;
b) man amplifiziert die DNA Probe, wobei man ein fluoreszenzmarkiertes dCTP-Derivat zusetzt;
c) man hybridisiert die amplifizierte DNA Probe an ein oder mehrere immobilisierte Oligomere, wobei die immobilisierten Oligomere jeweils komplementär zu mindestens einem der im Amplifikationsschritt verwendeten Primer sind; und
d) man misst die Fluoreszenz der hybridisierten Amplifikate quantitativ.

Erfindungsgemäß bevorzugt ist es, dass man in Schritt a) als Reagenz eine Bisulfitlösung verwendet.

Weiterhin bevorzugt ist es, dass man in Schritt b) für die Amplifikation PCR einsetzt.

Erfindungsgemäß besonders bevorzugt ist es, dass in Schritt b) das fluoreszenzmarkierte dCTP-Derivat Cy3-dCTP oder Cy5-dCTP ist.

Weiterhin ist es erfindungsgemäß besonders bevorzugt, dass man die Fluoreszenzfarbstoffe Cy3 und/oder Cy5 als Markierung verwendet.

Erfindungsgemäß bevorzugt ist es ferner, dass man für die Hybridisierung der Amplifikate in Schritt c) ein Array von zu den Primern aus Schritt b) komplementären Oligomeren verwendet.

Weiterhin bevorzugt ist es, dass man in Schritt b) die Amplifikation von mehreren DNA Abschnitten gleichzeitig durchführt.

Besonders bevorzugt ist es, dass man die in Schritt d) gemessenen Werte mit der Fluoreszenz anderer, analog behandelter DNA-Proben abgleicht und dadurch Information über den relativen Methylierungsgrad unterschiedlicher Gewebe oder Zellproben erhält.

Die Erfindung beschreibt somit ein Verfahren zur relativen Quantifizierung der Cytosin Methylierung in DNA Proben. Die DNA Proben werden chemisch so behandelt, dass Cytosin und Methylcytosin unterschiedlich reagieren und nur Methylcytosin-Positionen ihr Basenpaarungsverhalten beibehalten. Nachfolgend wird die DNA amplifiziert, wobei das verwendete Cytosintriphosphat mit einer Fluoreszenzmarkierung versehen ist. Die Amplifikate werden durch Hybridisierung an ein zu mindestens einem Primer komplementäres Oligomer gebunden, und die Festphase mehrfach gewaschen. Die Fluoreszenz am Immobilisierungsort gibt nun Aufschluss über die relative Anzahl der Cytosin Methylierungen im betreffenden amplifizierten DNA Abschnitt im Vergleich zu anderen analog behandelten Proben. Besonders bevorzugt erfolgt die Hybridisierung mehrerer unterschiedlicher Amplifikate einer Probe an einen Oligomer Array, dessen Fluoreszenzmuster nunmehr Aufschluß über das Methylierungsmuster in der DNA Probe gibt. Die Muster unterschiedlicher Proben werden abgeglichen.

Die Amplifikation von DNA mittels PCR ist Stand der Technik. Besonders bevorzugt ist der erfindungsgemäße Einsatz von fluoreszenzmarkierten Nukleotiden für PCR. Damit ist es vor allem möglich, ohne eine relativ teure Fluoreszenzmarkierung der Primer zugleich mehrere Fluorophore in ein Amplifikat einzuführen. Cy5-dCTP (Cy5 ist ein kommerziell erhältlicher Fluoreszenzfarbstoff) ist von der Firma Pharmacia erhältlich.

Mit anderen Worten, die Erfindung beschreibt also ein Verfahren zur relativen Quantifizierung der Cytosin Methylierung in DNA Proben. Die genomischen DNA Proben werden chemisch zunächst so behandelt, dass Cytosin und Methylcytosin unterschiedlich reagieren und nur Methylcytosin-Positionen ihr Basenpaarungsverhalten beibehalten. Bevorzugt wird dabei die Behandlung mit einer Bisulfitlösung, die fast ausschließlich mit den Cytosin Nukleobasen reagiert und diese nach alkalischer Hydrolyse in Uracil überführt. 5-Methylcytosin reagiert unter den gleichen Bedingungen nicht. Die Umwandlung von C in U führt an den nicht methylierten Positionen zu einer Veränderung der Basensequenz. Nachfolgend wird die DNA amplifiziert, wobei das verwendete Cytosintriphosphat mit einer Fluoreszenzmarkierung versehen ist. Bevorzugt wird hier Cy5-dCTP (Pharmacia) verwendet. Nur an den Positionen, an denen keine Umwandlung von C in U stattgefunden hat, kann in der PCR ein Cy5-C und damit eine Fluoreszenzmarkierung eingebaut werden. Damit ist die Anzahl der eingebauten Cy5-dCTPs in guter Näherung proportional zum Ausmaß der Methylierung im amplifizierten DNA Abschnitt. Nun werden auch in den Gegenstrang Cy5-Cs eingebaut, dort, wo sich im Bisulfit-behandelten Strang Guanin befunden hat. Dieser Einbau ist für die Fluoreszenzdetektion im Prinzip störend. Dieses Problem wird bei der vorliegenden Erfindung dadurch umgangen, dass nur relevante Einzelstränge aus der Lösung heraus an eine Festphase gebunden werden. Nach einem thermischen Denaturierungsschritt werden sie durch Hybridisierung an ein zu mindestens einem Primer komplementäres und immobilisiertes Oligomer gebunden, und die Festphase anschließend mehrfach gewaschen. Die Intensität der Fluoreszenz am Immobilisierungsort gibt nun Aufschluß über die relative Anzahl der Cytosin Methylierungen im betreffenden amplifizierten DNA Abschnitt im Vergleich zu anderen analog behandelten Proben. Besonders bevorzugt erfolgt die Hybridisierung mehrerer unterschiedlicher Amplifikate einer Probe an einen Oligomer Array, dessen Fluoreszenzmuster nunmehr Aufschluß über das Methylierungsmuster in der DNA Probe gibt. Der Oligomer Array wird bevorzugt durch das Aufbringen separat synthetisierter Oligomere auf einen Träger (Chip) oder durch photolithographische Techniken hergestellt (Stand der Technik). Das Trägermaterial ist bevorzugt durch Silanisierung derivatisiertes Glas.

Die Fluoreszenzmuster unterschiedlicher Proben werden in eine Datenbank eingegeben und abgeglichen. Besonders bevorzugt ist die Verwendung des Verfahrens für die Gewinnung von Information über den relativen Methylierungsgrad unterschiedlicher Gewebe eines Individuums und gleicher Gewebe verschiedener Individuen.

Das folgende Ausführungsbeispiel erläutert die Erfindung:

### Beispiel 1:

### Kalibrierung des Verfahrens zur relativen Quantifizierung von Cytosin-Methylierung

Das folgende Beispiel für ein Verfahren zur relativen Quantifizierung der Methylierung von Cytosin Basen in amplifizierten Nukleinsäurefragmenten bezieht sich auf Fragmente des Multi Drug Resistance (MDR1) und Multi Resistance Protein (MRP3) Gens.

Im ersten Schritt werden Glassubstrate chemisch modifiziert, so dass eine gezielte Anbindung von Oligonukleotiden erfolgen kann, wie es Stand der Technik ist. Durch Aufbringen von verschiedenartigen Oligonukleotiden auf ein Substrat lassen sich üblicherweise Oligonukleotid-Arrays herstellen. Dazu werden die Substrate silanisiert, wobei das Silan eine funktionalisierte Alkylkette trägt.

Anschließend wird die Oberfläche mit einem bifunktionalen Linker versehen, beipielsweise Phenylendiisothiocyanat oder Adipinsäuredi (N-hydroxysuccinimidyl) ester. Dieser Linker erlaubt eine kovalente Anbindung der Oligonukleotide unter basischen Bedingungen. In diesem Fall werden die Oligonukleotide 
AAC TCC CCA ATA CTA CAA CC (MRP3),
AAAATACACAAACRCTCCCA (MRP3) und
CTACAATAATCTTTCTTCAACATACTTA (MDR1),
TAA AAA CTA TCC CAT AAT AAC TCC CAA C (MDR1),
 die komplementär zu den im Amplifikationsschritt verwendeten Primern sind, durch automatisches Pipettieren oder Spotten in definierten Positionen auf die Substratoberfläche gebracht.

Zur relativen Quantifizierung der Methylierung von Cytosin Basen in Nukleinsäurefragmenten werden je zwei aufmethylierte bzw. unmethylierte Nukleinsäurefragmente (je MDR1 und MRP3), mit deren Hilfe unbekannt methylierte Proben verglichen werden können, zur Kalibrierung verwendet.

### Beispiel 2:

### Herstellung der unmethylierten Referenzprobe

Im Falle der unmethylierten Probe setzt man eine genomische DNA-Probe (18 ng), die mit dem Restriktionenzym Mss1 verdaut wurde, ein. Die erste Probe wird unter Verwendung von je 25 pmol spezifischen Primern 
CAAGCATGCTGAAGAAAGACCACTGCAG (MDR1),
TGGGAACTGTCCCATAATAACTCCCAAC (MDR1)
 und folgendem Programm: T=96.0 °C 10 min; T=96.0 °C 30 s; T=58.0 °C 1:15 min; T=72.0 °C 2 min; T=72.0 °C 15 min über 40 Cyclen amplifiziert.

Die zweite Probe wird ebenfalls mit 25 pmol spezifischen Primern 
GGC TGC AGC ACT GGG GAG CC (MRP3),
GGC TCC CCA GTG CTG CAG CC (MRP3)
 und folgendem Programm: T=96.0 °C 10 min; T=96.0 °C 1 min; T=55.0 °C 45 s; T=72,0 °C 1:15 min über T=72,0 °C 10 min über 45 Cyclen amplifiziert. Beide Amplifikate werden mit Bisulfit (=Hydrogensulfit, Disulfit) und einem Radikalfänger bei erhöhter Temperatur chemisch umgewandelt. Die Bisulfit Reaktion führt zur Umwandlung aller nicht methylierten Cytosinbasen in Uracil. Zur Reinigung der modifizierten Amplifikate werden diese auf eine Reversed Phase C18 Festphase gebunden und durch Waschen von Chemikalien befreit. Anschließend wird die DNA mit einem polaren Lösungsmittel wie z. B. Acetonitril oder Wasser eluiert. Die alkalische Hydrolyse der mit Bisulfit behandelten Amplifikate erfolgt unmittelbar vor der erneuten spezifischen Amplifikation bei der das fluoreszenzmarkierte Nukleotid eingesetzt wird. Es werden definierte Fragmente der Länge 633 bp (MDR1) und 640 bp (MRP3) amplifiziert, die durch den definierten Einbau von Cy5-dCTP fluoreszieren.

Die Amplifikationen der beiden Gene MDR1 und MRP3 werden wiederum mit je 25 pmol Primer 
TAAGTATGTTGAAGAAAGATTATTGTAG (MDR1),
TAAAAACTATCCCATAATAACTCCCAAC(MDR1),
AACTCCCCAATACTACAAC (MRP3),
TGGGAGYGTTTGTGTATTTT (MRP3)
 und 0.5-0.75 mM Cy5-dCTP in der PCR durchgeführt. Die PCR läuft unter folgenden Bedingungen ab. MDR1: T=96.0 °C 20 min; T=96.0 °C 30 s; T=54.6 °C 1:15 min; T=72,0 °C 2 min; T=72.0 °C 15 min über 40 Cyclen, MRP3: T=96.0 °C 20 min; T=96.0 °C 30 s; T=61.70 °C 1:15 min, T=72,0 °C 2 min; T=72,0 °C 15 min über 40 Cyclen.

### Beispiel 3:

### Herstellung der methylierten Referenzprobe

Für die Aufmethylierung von genomischer DNA inkubiert man 1µg DNA bei 37 °C für eine Stunde mit 1 unit der Methylase Sss1. Das Enzym wird anschließend deaktiviert. Die methylierte Probe wird Mss1 verdaut und spezifisch amplifiziert.

Beide Amplifikate werden mit Bisulfit (=Hydrogensulfit, Disulfit) und einem Radikalfänger bei erhöhter Temperatur chemisch umgewandelt. Die Bisulfit Reaktion führt zur Umwandlung aller nicht methylierten Cytosinbasen in Uracil. Zur Reinigung der modifizierten Amplifikate werden diese auf eine Reversed Phase C18 Festphase gebunden und durch Waschen von Chemikalien befreit. Anschließend wird die DNA mit einem polaren Lösungsmittel wie z. B. Acetonitril oder Wasser eluiert. Die alkalische Hydrolyse der mit Bisulfit behandelten Amplifikate erfolgt unmittelbar vor der erneuten spezifischen Amplifikation bei der das fluoreszenzmarkierte Nukleotid eingesetzt wird. Es werden definierte Fragmente wie unter Beispiel 2 amplifiziert.

Die PCR wird mit 25 pmol je spezifischem Primer 
TAAGTATGTTGAAGAAAGATTATTGTAG (MDR1),
TAAAAACTATCCCATAATAACTCCCAAC(MDR1),
AACTCCCCAATACTACAAC (MRP3),
TGGGAGYGTTTGTGTATTTT (MRP3)
 und 0.5-0.75 mM Cy5-dCTP durchgeführt.

### Beispiel 4:

### Hybridisierung der Amplifikate

Die Amplifikate werden durch Hybridisierung in an sich bekannter Weise an den entsprechenden, den nicht Cytosin enthaltenden Primern komplementären oberflächengebundenen Oligomeren festgehalten und die Festphase mehrfach gewaschen, um nicht komplementäre Amplifikate zu entfernen.

Die an die oberflächengebundenen Oligonukleotide (Oligomerarray), das sind in diesem Beispiel wie unter 1) die Sequenzen 
AAC TCC CCA ATA CTA CAA CC (MRP3),
AAAATACACAAACRCTCCCA (MRP3) und
CTACAATAATCTTTCTTCAACATACTTA (MDR1),
TAA AAA CTA TCC CAT AAT AAC TCC CAA C (MDR1),
 gebundenen Amplifikate werden anhand ihrer Fluoreszenz bei 635 nm nachgewiesen. Dazu wird ein handelsüblicher Fluoreszenzscanner (z. B: Genepix 4000, Axon Laboratories) verwendet.

### Beispiel 5:

### Auswertung

Nach der Kalibrierung des Systems mit ursprünglich aufmethylierten und unmethylierten Referenzproben können nun weitere Proben gemessen werden. Liegen wesentliche Unterschiede in der Methylierung der verschiedenen Amplifikate der zu untersuchenden Proben vor, die entsprechend den nicht aufmethylierten Proben, aber ohne eine vorherige Amplifikation der genomischen DNA, aufbereitet werden, so fällt auf, dass bestimmte Punkte auf dem Array relativ zu den anderen eine deutlich erhöhte oder verminderte Fluoreszenz zeigen, sofern die Amplifikate in den einzelnen zu untersuchenden Proben in vergleichbarer Konzentration vorliegen.

Das Problem der vergleichbaren Konzentrationen, das dazu führt, dass meist auf diese Weise nur relativ extreme Unterschiede erfasst werden (was allerdings im Falle einer nahezu vollständigen Aufmethylierung eines CpG islands gegeben ist), lässt sich durch die Fluoreszenzmarkierung der Primer mit einem anderen Farbstoff umgehen (z.B. Cy3). In diesem Fall wird die Probenaufbereitung genau gleich, nur mit Cy3-gelabelten Primern, durchgeführt. Die Intensitäten bei 532 nm (Cy3) an den einzelnen Punkten dienen dann zunächst zum Abgleich der Konzentrationen der einzelnen Amplifikate, bevor die relative Quantifizierung der methylierten Cytosine bei 635 nm (Cy5) in den einzelnen Amplifikaten vorgenommen wird. Die Cy3-Werte dienen dabei als Korrekturfaktor. Die Art und Weise der Auswertung von Fluoreszenzmessungen ist dem Fachmann bekannt.

## Patentansprüche

1. Verfahren zur relativen Quantifizierung der Methylierung von Cytosin Basen in DNA-Proben, **dadurch gekennzeichnet, dass** man folgende Verfahrensschritte ausführt:
a) man setzt eine genomische DNA-Probe mit einem Reagenz chemisch um, wobei 5-Methylcytosin und Cytosin unterschiedlich reagieren und diese somit nach der Reaktion ein unterschiedliches Basenpaarungsverhalten in der DNA Duplex zeigen;
b) man amplifiziert die DNA Probe, wobei man ein fluoreszenzmarkiertes dCTP-Derivat zusetzt;
c) man hybridisiert die amplifizierte DNA Probe an ein oder mehrere immobilisierte Oligomere, wobei die immobilisierten Oligomere jeweils komplementär zu mindestens einem der im Amplifikationsschritt verwendeten Primer sind; and
d) man mißt die Fluoreszenz der hybridisierten Amplifikate quantitativ.

2. Verfahren nach Anspruch 1, **dadurch gekennzeichnet, dass** man in Schritt a) als Reagenz eine Bisulfitlösung verwendet.

3. Verfahren nach einem der Ansprüche 1 oder 2, **dadurch gekennzeichnet, dass** man in Schritt b) für die Amplifikation PCR einsetzt.

4. Verfahren nach einem der vorangehenden Ansprüche, **dadurch gekennzeichnet, dass** in Schritt b) das fluoreszenzmarkierte dCTP-Derivat Cy3-dCTP oder Cy5-dCTP ist.

5. Verfahren nach einem der Ansprüche 1 bis 3, **dadurch gekennzeichnet, dass** man die Fluoreszenzfarbstoffe Cy3 und/oder Cy5 als Markierung verwendet.

6. Verfahren nach einem der vorangehenden Ansprüche, **dadurch gekennzeichnet, dass** man für die Hybridisierung der Amplifikate in Schritt c) ein Array von zu den Primern aus Schritt b) komplementären Oligomeren verwendet.

7. Verfahren nach einen der vorangehenden Ansprüche **dadurch gekennzeichnet, dass** man in Schritt b) die Amplifikation von mehreren DNA Abschnitten gleichzeitig durchführt.

8. Verfahren nach Anspruch 1, wobei man die in Schritt d) gemessenen Werte mit der Fluoreszenz anderer, analog behandelter DNA-Proben abgleicht und dadurch Information über den relativen Methylierungsgrad unterschiedlicher Gewebe oder Zellproben erhält.

## Claims

1. A method for the relative quantification of the methylation of cytosine bases in DNA samples, **characterized in that** the following method steps are conducted:
a) a genomic DNA sample is chemically reacted with a reagent, wherein 5-methylcytosine and cytosine react differently and these thus show a different base pairing behavior in the DNA duplex after the reaction;
b) the DNA sample is amplified, whereby a fluorescently labeled dCTP derivative is added;
c) the amplified DNA sample is hybridized to one or more immobilized oligomers, whereby the immobilized oligomers are each complementary to at least one of the primers used in the amplification step; and
d) the fluorescence of the hybridized amplified products is quantitatively measured.

2. The method according to claim 1, further **characterized in that** a bisulfite solution is used in step (a) as the reagent.

3. The method according to one of claims 1 or 2, further **characterized in that** PCR is used in step (b) for the amplification.

4. The method according to one of the preceding claims, further **characterized in that** in step (b) the fluorescently labeled dCTP derivative is Cy3-dCTP or Cy5-dCTP.

5. The method according to one of claims 1 to 3, further **characterized in that** the fluorescent dyes Cy3 and/or Cy5 are used as the label.

6. The method according to one of the preceding claims, further **characterized in that** an array of oligomers complementary to the primers of step (b) is used for the hybridizing of the amplified products in step (c).

7. The method according to one of the preceding claims, further **characterized in that** the amplification of several DNA segments in step (b) is conducted simultaneously.

8. The method according to claim 1, wherein the values measured in step (d) are equilibrated with the fluorescence of other, analogously treated DNA samples and in this way information is obtained on the relative degree of methylation of different tissues or different cell samples.

## Revendications

1. Procédé de quantification relative de la méthylation de bases de cytosine dans des échantillons d'ADN, **caractérisé en ce que** l'on réalise les étapes de procédé suivantes :
a) on traite un échantillon d'ADN génomique avec un réactif au plan chimique, la 5-méthylcytosine et la cytosine réagissant différemment et présentant ainsi un comportement d'appariement de bases différent après la réaction dans le duplex d'ADN ;
b) on amplifie l'échantillon d'ADN, en employant un dérivé de dCTP marqué par fluorescence ;
c) on hybride l'échantillon d'ADN amplifié sur un ou plusieurs oligomères immobilisés, les oligomères immobilisés étant à chaque fois complémentaires d'au moins une amorce utilisée dans l'étape d'amplification ; et
d) on mesure la fluorescence des amplificats hybridés de façon quantitative.

2. Procédé selon la revendication 1, **caractérisé en ce qu'**on utilise dans l'étape a) une solution de bisulfite comme réactif.

3. Procédé selon l'une des revendications 1 ou 2, **caractérisé en ce qu'**on emploie dans l'étape b) une PCR pour l'amplification.

4. Procédé selon l'une quelconque des revendications précédentes, **caractérisé en ce que** le dérivé de dCTP marqué par fluorescence est Cy3-dCTP ou Cy5-dCTP dans l'étape b).

5. Procédé selon l'une quelconque des revendications 1 à 3, **caractérisé en ce qu'**on utilise la substance fluorescente Cy3 et/ou Cy5 comme marquage.

6. Procédé selon l'une des revendications précédentes, **caractérisé en ce qu'**on utilise un ensemble d'oligomères complémentaires des amorces de l'étape b) pour l'hybridation des amplificats de l'étape c).

7. Procédé selon l'une quelconque des revendications précédentes, **caractérisé en ce qu'**on réalise simultanément l'amplification de plusieurs portions d'ADN dans l'étape b).

8. Procédé selon la revendication 1, dans laquelle on compare les valeurs mesurées à l'étape d) avec la fluorescence d'autres échantillons d'ADN traités de façon analogue et on obtient ainsi des informations sur le degré de méthylation relatif de divers tissus ou échantillons cellulaires.
